# EUROPEAN PATENT APPLICATION

(11) **EP 3 241 581 A1**
(43) Date of publication of application: **08.11.2017**
(21) Application number: 16168070.7
(22) Date of filing: 03.05.2016
(51) Int. Cl.: A61M 5/20, A61M 5/24

(54) **NON-RELEASABLE SNAP CONNECTION OF CARTRIDGE HOLDER TO SYRINGE**

(71) Applicant: Sanofi-Aventis Deutschland GmbH, 65929 Frankfurt (DE)
(72) Inventor: The designation of the inventor has not yet been filed

(57) **Abstract**

The present invention relates in one aspect to a housing for an injection device, comprising:
- a body (30) to accommodate a drive mechanism (5) to operably engage with a piston (52) of a cartridge (50) filled with a liquid injectable medicament (53),
- a cartridge holder (20) having a cartridge receiving space (25) to accommodate the cartridge (50),
- wherein the cartridge holder (20) and the body (30) comprise an elongated shape and extend along an axial longitudinal direction (1, 2),
- wherein the cartridge holder (20) has an insert section (21) at a proximal end (23),
- wherein the body (30) has a receptacle (31) at a distal end (33) to receive the insert section (21) of the cartridge holder (20),
- wherein the body (30) comprises a first latch element (34) on an inside (32) of a sidewall (32) of the receptacle (31),
- wherein the cartridge holder (20) comprises a first through recess (24) in a sidewall (22) of the insert section (21) which through recess (24) is configured to receive the first latch element (34), wherein the through recess (24) comprises a narrowed neck receiving portion (24a) extending axially into a widened latch receiving portion (24b), wherein a first lateral width (w1) of the neck receiving portion (24a) is smaller than a second lateral width (w2) of the latch receiving portion (24b).

## Description

The present invention relates in one aspect to a housing of an injection device, such like a pen-type injector which is operable for setting and dispensing of a dose of a medicament. In particular, the invention relates to an interconnection of two housing components of an injection device

### Background and Prior Art

injection devices for setting and dispensing a single or multiple doses of a liquid medicament are as such well-known in the art. Generally, such devices have substantially a similar purpose as that of an ordinary syringe.

injection devices, in particular pen-type injectors have to meet a number of user-specific requirements. For instance, with patient's suffering chronic diseases, such like diabetes, the patient may be physically infirm and may also have impaired vision. Suitable injection devices especially intended for home medication therefore need to be robust in construction and should be easy to use. Furthermore, manipulation and general handling of the device and its components should be intelligible and easy understandable. Moreover, a dose setting as well as a dose dispensing procedure must be easy to operate and has to be unambiguous.

Typically, such devices comprise a housing including a particular cartridge holder, adapted to receive a cartridge at least partially filled with the medicament to be dispensed. Such devices further comprise a drive mechanism, usually having a displaceable piston rod which is configured to operably engage with a piston of the cartridge. By means of the drive mechanism and its piston rod, the piston of the cartridge is displaceable in a distal direction or dispensing direction and may therefore expel a predefined amount of the medicament via a piercing assembly, which is in fluid communication with an interior of the cartridge and which is configured to be coupled to a distal end section of the housing of the injection device.

The medicament to be dispensed by the injection device is provided and contained in a multidose cartridge. Such cartridges typically comprise a vitreous barrel sealed in distal direction by means of a pierceable seal and being further sealed in proximal direction by the piston. With reusable injection devices an empty cartridge is replaceable by a new one. In contrast to that, injection devices of disposable type are to be discarded when the medicament in the cartridge has been dispensed or used-up.

Document WO 2008/003560 A1 describes for instance a pre-filled injection device having a container and a dose setting mechanism encapsulated inside a housing. The housing is made from two parts which are locked together by a bayonet coupling. In addition to the bayonet coupling, the two parts are also provided with irreversible locking means locking the two parts permanently together.

During a final assembly process of such handheld injection devices a cartridge containing the injectable medicament is positioned and assembled inside a distal housing component forming the cartridge holder. A drive mechanism including at least a piston rod to operably engage with the piston of the cartridge is assembled inside a proximal housing component that forms or constitutes a body. The cartridge holder with the cartridge assembled therein forms a cartridge holder preassembly whereas the body with the drive mechanism assembled therein forms or constitutes a body preassembly.

During a final step of assembly the cartridge holder preassembly and the body preassembly are assembled together. For this a proximal end of the cartridge holder is fixed to a distal end of the body. Typically, cartridge holder and body comprise an insert section and a correspondingly-shaped receptacle so that the proximal end of the cartridge holder and the distal end of the body are assembled in a nested or interleaved way. In a mutually overlapping section there are typically provided positively engaging locking means, such like radially outwardly or radially inwardly extending mutually corresponding snap features. For instance, an insert section of the cartridge holder may be provided with at least one or several through holes in a sidewall section that are configured to receive and to engage with at least one radially inwardly protruding latch element provided on an inside facing portion of a sidewall of the receptacle of the body, in which the insert section of the cartridge holder is located.

Such a clip connection based on snap features and holes may be disadvantageous for a barrel of the cartridge made from a vitreous material. The cartridge firmly attached or assembled inside the cartridge holder may experience a non-neglectable radially inwardly directed compression as the snap features of the receptacle of the body compress a portion of the cartridge holder radially inwardly before they snap into correspondingly-shaped holes in the insert section of the cartridge holder. In order to establish such clip connections the sidewall of interleaved housing sections of cartridge holder and body are temporally subject to a radial deformation. Even though such radial deformations of the cartridge holder are rather marginal they may cause significant stress to the glass cartridge assembled therein.

It is therefore an object of the present invention to improve the mechanical connection between housing components of a handheld injection device, such as an injection pen, that provides a reliable and stable fixing of the housing components without exerting significant stress to the glass cartridge during assembly. A mechanical stress level applied to the cartridge should be at least drastically reduced compared to prior art solutions. It is a further aim to provide a non-detachable or irreversible connection of two housing components of an injection device, which is robust and long-lasting and which is configured to withstand external mechanical forces.

### Summary

In one aspect a housing for an injection device, in particular for a pen-type injection device is provided. The housing comprises a body to accommodate a drive mechanism to operably engage with a piston of a cartridge. The cartridge is typically filled with a liquid injectable medicament. The housing further comprises a cartridge holder to accommodate the cartridge. The cartridge holder typically has a cartridge receiving space that is completely captured or taken by the cartridge when assembled inside the cartridge holder. In a final assembly configuration the cartridge receiving space coincides with the outer circumference and with the outer geometry of the cartridge assembled therein.

The cartridge holder and the body are of elongated shape and extend along an axial direction. The cartridge holder and the body may be of substantially tubular or cylindrical shape. The axial direction may thus coincide with the longitudinal axis of the tubular or cylindrically-shaped cartridge holder or with the co-aligned body. The cartridge holder further has an insert section at a proximal end and the body has a receptacle at a distal end to receive the insert section of the cartridge holder. In a final assembly configuration the body and the cartridge holder are aligned substantially parallel with their longitudinal axes and the insert section of the cartridge holder is located and fitted inside the receptacle of the body. Hence, the outer diameter of the insert section matches with the inner diameter of the receptacle. In this way cartridge holder and body are assembled in a nested or interleaved way in which the receptacle and the insert section of body and cartridge holder mutually overlap in a radial direction.

The body comprises a first latch element on an inside of a sidewall of the receptacle and the cartridge holder comprises a first through recess in a sidewall of the insert section. The through recess is configured to receive the first latch element when cartridge holder and body arrive in a final assembly configuration. The through recess comprises a narrowed neck receiving portion extending axially into a widened latch receiving portion. A first width of the neck receiving portion is smaller than a second width of the latch receiving portion. As seen in an axial direction and as the neck receiving portion extends axially into the latch receiving portion, the width of the through recess widens, typically in transverse or tangential direction when assuming a substantially cylindrically-shaped body or cartridge holder.

The first latch element and the first through recess are configured to form a clip connection or a snap fit connection of cartridge holder and body. The first latch element and the first through recess are configured to provide a positive interlock of cartridge holder and body. Typically but not necessarily the first latch element and the first through recess are configured to establish and to provide a non-releasable engagement of cartridge holder and body. Once assembled cartridge holder and body cannot be detached or released without substantially destroying at least one of the cartridge holder and the body. The positive engagement of the cartridge holder and the body as provided by the at least first latch element and the at least one through recess is of permanent type or of non-releasable type.

The neck receiving portion and the latch receiving portion of the first through recess form a kind of an indentation or undercutting as the first latch element is introduced in axial direction into said first through recess. The indentation or the undercutting are located in the sidewall of the cartridge holder and extend in a tangential or circumferential direction.

Typically, the first latch element extends inwardly from the inside of the sidewall of the receptacle. The radial or inwardly directed extension of the first latch element substantially matches with the thickness of the sidewall of the insert section of the cartridge holder. Hence, in a final assembly configuration the first through recess of the cartridge holder may be completely occupied by the first latch element. The outer or circumferential geometry of the first latch element matches with an inner geometry of the first through recess. Since the first width of the neck receiving portion is smaller than the second width of the latch receiving portion and since the first through recess is configured to receive the first latch element the sidewall of the insert section of the cartridge holder may be predominantly subject to a temporary and flexible deformation in tangential or lateral direction with regard to the tubular elongation of the cartridge holder.

For establishing of a positive interlock of cartridge holder and body inevitable radial deformations or inwardly directed deformations of the cartridge holder can be remarkably reduced compared to solutions as they are known in the prior art. In effect, mechanical load applied to the cartridge during assembly and during a mutual fixing of cartridge holder and body can be further reduced. A danger or likelihood of damaging the cartridge during a final assembly process of the housing or of the injection device can be reduced. At the same time a durable, reliable and non-releasable interconnection of body and cartridge holder can be provided.

According to an embodiment the first through recess adjoins the proximal end of the cartridge holder. The through recess is further open towards the proximal end of the cartridge holder. In other words, the first through recess is a kind of a slit extending from a proximal end of the cartridge holder, hence from a proximal end of the cartridge holder's insert section towards the distal direction. Since the first through recess adjoins the proximal end a sidewall section of the insert section adjoining the first through recess becomes flexible in a tangential direction. This enables generation of a positive interconnection of the first latch element and the first through recess only by way of a flexible deformation of the insert section of the cartridge holder in tangential direction. In this way a degree of radially inwardly directed load acting on a cartridge located inside the cartridge holder can be further reduced or even entirely eliminated.

According to another embodiment the neck receiving portion of the first through recess adjoins the proximal end of the insert section. The neck receiving portion is further located axially between the latch receiving portion and the proximal end of the insert section. The neck receiving portion with a rather limited first width may form a rather constricted opening or a kind of a bottleneck through which the first latch element is to be urged in distal direction in order to arrive in the latch receiving portion. Typically, in a transition area between the neck receiving portion and the latch receiving portion the first through recess comprises at least one abutment face facing in distal direction so as to engage with a correspondingly-shaped abutment face of the first latch element facing in proximal direction. Said abutment faces of the first latch element and the first through recess may axially engage as the insert section of the cartridge holder has been fully received inside the receptacle of the body thus forming at least a portion of an axial interlock of cartridge holder and body.

In another embodiment the first latch element comprises a narrowed neck portion extending axially into a widened latch portion. The neck portion of the first latch element corresponds and substantially coincides with the neck receiving portion of the first through recess. Also the latch portion of the first latch element corresponds to or coincides with the latch receiving portion of the first through recess. In this way and as a final assembly configuration of cartridge holder and body has been reached the first latch element may entirely occupy the first through recess.

In a further embodiment the latch portion comprises a maximal width that is larger than the first width of the neck receiving portion of the first through recess. The first width, the second width as well as the maximal and a minimal width of the first through recess and of the latch portion is measured in tangential, circumferential or lateral direction, i.e. perpendicular to the axial or longitudinal direction of the body or cartridge holder but along the extension of the sidewalls of cartridge holder and body, respectively.

Since the maximal width of the latch portion is larger than the first width of the neck receiving portion and since the latch portion and the latch receiving portion as well as the neck receiving portion are located at a common radial distance from a center axis of the cartridge holder or body and since the outer circumference or the outer diameter of the insert section substantially matches with the inner circumference or inner diameter of the receptacle of the body a positive engagement of the first latch element with the first through recess is attainable only by way of a sliding and translational movement of the insert section into the receptacle in proximal direction.

Due to the above mentioned geometries of maximal width and first width of latch portion and neck receiving portion at least one of the first latch elements and the sidewall of the cartridge holder will be subject to a temporary elastic deformation during the assembly process. As the first latch element is urged axially into the first through recess the narrowed neck portion of the first through recess will be subject to a widening in tangential direction, hence perpendicular to the axial or longitudinal direction but not in radial direction. It is particularly the cartridge holder in the region of its first through recess that is subject to a tangential elastic deformation during the assembly process of cartridge holder and body. In this way the degree of an elastic deformation in radial direction can be substantially reduced or entirely eliminated, thereby reducing the mechanical load that may be otherwise applied to the vitreous barrel of the cartridge.

According to another embodiment the latch portion of the first latch element is wedge-shaped in tangential direction with an increasing width in proximal direction. Hence, a distal end of the first latch element facing towards the cartridge holder has a smaller width than a proximal end of the first latch element. By means of a wedge-shaped geometry the first latch element is operable to continuously and smoothly widen the narrowed neck receiving portion of the first through recess during the process of assembly. Once a final assembly configuration has been reached the wedge-shaped first latch element entirely occupies the correspondingly-shaped latch receiving portion of the first through recess. The narrowed neck portion of the first latch element may entirely occupy the correspondingly-shaped neck receiving portion of the first through recess. In this way and once the latch element completely occupies the first through recess the mutually corresponding geometric structures of the latch element and the first through recess effectively inhibit any rotational motion of the cartridge holder relative to the body.

Typically, the first latch element is symmetric with regard to the axial longitudinal direction. It comprises a wedge-shaped geometry towards both lateral or tangential faces. In this way, the effect of tangentially widening the narrowed neck receiving portion of the first through recess can be improved.

According to another embodiment the latch portion comprises a minimal width at a distal end and further comprises the maximal width at a proximal end. The minimal width of the latch portion is typically smaller than the first width of the neck receiving portion of the first through recess. In this way at least the distal end of the latch portion is insertable into the neck receiving portion of the first through recess without any deformation of the cartridge holder or body. The distal end of the latch portion is insertable in distal direction into the neck receiving portion which is open towards the proximal direction. During the assembly process and during the further movement of the latch element into the first through recess and into the neck receiving portion the wedge-shaped side faces of the first latch element will get in abutment with oppositely located sidewall sections of the cartridge holder that confine the neck receiving portion.

As the latch element is urged further into the first through recess in distal direction the wedge-shaped side faces of the first latch element will start to widen the neck receiving portion of the first through recess in tangential direction due to the continuously increasing width of the wedge-shaped latch element, in particular due to the wedge-shaped geometry of the latch portion thereof.

According to a another embodiment the receptacle comprises a first protrusion in addition to the first latch element. The first protrusion is axially elongated and extends inwardly from the sidewall of the receptacle at a first distance from the first latch element. As seen in axial or longitudinal direction the first protrusion and the first latch element may be located at the same axial position. They are separated along a tangential or circumferential direction of the e.g. tubular-shaped sidewall of the receptacle. The first protrusion is configured to prevent disengagement of body and cartridge holder once these components are mutually assembled.

In another embodiment the insert section comprises a first slit adjoining the proximal end of the insert section and extends in distal direction from the proximal end of the cartridge holder. The first slit is located at the first distance from the first through recess. It is therefore configured to receive the first protrusion on the inside of the receptacle of the body. The first slit as well as the first protrusion may be rather straight-shaped. A tangential or lateral width of the first slit may exactly match with a tangential or lateral width of at least a portion of the first protrusion. Once the cartridge holder and the body are in a final assembly configuration the first protrusion may at least occupy a portion of the first slit in tangential direction. In this way, the first protrusion and the first slit provide a rotational interlock of cartridge holder and body with regard to a rotation axis coinciding with the longitudinal axial direction of the cartridge holder or the body.

Since the tangential distance between the first through recess and the first slit is identical to the tangential distance between the first protrusion and the first latch element the first through recess and the latch element as well as the first slit and the first protrusion simultaneously engage during insertion of the insert section of the cartridge holder into the receptacle of the body. Furthermore, the first slit engaging with the first protrusion provides an axial guiding function during insertion of the insert section into the receptacle of the body.

The radial extension of the first protrusion may be substantially identical to the width of thickness of the sidewall of the insert section. Likewise the first through recess the first slit is also configured as a through opening in the sidewall of the insert section. The first slit is also open towards the proximal end so as to receive the first protrusion extending in distal direction along the inside of the sidewall of the receptacle.

In a further embodiment a first sidewall section of the insert section, which is confined by the first through recess and the first slit is flexible in a tangential direction from an initial unbiased state towards a biased state when the latch element actually enters into the first through recess. In the biased state the neck receiving portion of the first through recess is in engagement with the wedge-shaped latch portion of the first latch element. Consequently and since the sidewall of the insert section is subject to a tangentially directed deformation the first slit located at the predefined first distance from the first through recess will be subject to a temporary narrowing in tangential direction.

In the biased state in which the sidewall of the insert section is subject to an elastic deformation the first through recess tangentially widens at the expense of the first slit. In order to allow a tangential narrowing of the first slit, at least one of the first slit and the first protrusion or both may comprise a wedge-shaped geometry as seen in axial direction. Apart from that it is also conceivable that the first slit and the first protrusion are somewhat straight-shaped or rectangular-shaped. Then, and as will be explained later on, a tangential temporary flexing of the first sidewall section of the insert section may be accompanied by a well-defined but tiny radial deflection.

According to another embodiment a transverse size of the first protrusion is substantially equal to a transverse size of the first slit when the first sidewall section is in the unbiased state. The insert section will be in the unbiased or initial configuration prior to an assembly process as well as after a final assembly configuration has been reached. Since the transverse or tangential size or a maximal tangential width of the first protrusion exactly matches with the transverse size or tangential width of the first slit the mutual engagement of the first protrusion and the first slit provides an effective rotational interlock of cartridge holder and body when in the final assembly configuration.

It is generally conceivable, that the first protrusion has a minimal transverse size or tangential width at a distal end and comprises a maximal transverse size at or near a proximal end. In this way the widest portion of the first protrusion will just enter the first slit at the end of the assembly process of body and cartridge holder. That section of the first protrusion comprising a maximal tangential width may engage with the first slit substantially simultaneously with the completion of the snap-fit engagement of the first latch element with the first through recess.

In another embodiment the first protrusion further comprises a lateral abutment face to abut with a lateral abutment face of the first sidewall section of the insert section. The lateral abutment face of the first sidewall section confines the first slit. Typically, the first protrusion comprises two tangentially oppositely located lateral abutment faces having a lateral distance, .i.e. a distance in tangential direction, that substantially matches with the lateral distance between oppositely located lateral abutment faces confining the first slit in tangential or lateral direction. In this way and as a final assembly configuration is reached the first protrusion entirely occupies a lateral or tangential cross-section of the first slit of the insert section. Hence, oppositely located lateral abutment faces of the first protrusion get in direct abutment with lateral abutment faces laterally confining the first slit in the sidewall of the insert section. A tangential size or width of the first protrusion matches with and is substantially identical with a tangential or circumferential size or width of the slit provided in the insert section.

The first protrusion entirely occupying at least a portion of the first slit in tangential direction effectively prevents any further tangentially or laterally directed deformation of the first sidewall section of the insert section or even prevents any further tangential or lateral deflection of the insert section as a whole. In this way, the first protrusion engaged with and located inside the first insert section effectively prevents a release of the positive engagement of the first through recess and the latch element located therein.

According to another embodiment the first protrusion comprises a ramp section with a beveled surface extending inwardly from the inside of the sidewall of the receptacle as seen in proximal direction. The ramp section with its beveled surface facing in distal direction and having a smoothly and continuously increasing distance from the inside of the sidewall of the receptacle towards the proximal direction provides a small but distinct radially inwardly directed deflection of the first sidewall section of the insert section of the cartridge holder during the assembly process. Due to the biasing and/or tangentially directed deformation of the first sidewall section during insertion of the first latch element into the first through recess the lateral or tangential width of the first slit is inevitably narrowed compared to the initial unbiased state.

Since the first slit is only capable to receive the first protrusion when it is in the initial unbiased state the ramp section with its beveled surface provides a well-defined radial deflection at least of a tangential corner section of the first sidewall section. Here, at least that lateral edge of the first sidewall section engaging with the ramp section is subject to a slight but distinct radially inwardly directed deflection so as to radially overlap with the first protrusion during the assembly process. As soon as the final assembly configuration has been reached and when the latch portion of the first latch element engages with the latch receiving portion of the first through recess the first sidewall section and hence the first through recess will return into its initial configuration or into the unbiased state.

The first sidewall section will be subject to a tangential relaxation and the first slit will approach its initial unbiased state. The lateral or tangential width of the first slit will widen to such an extent that it is able to receive the first protrusion in its entirety. Then, the lateral abutment face of the first protrusion will laterally or tangentially abut with the lateral abutment face of the first sidewall section of the insert section. Once the first protrusion occupies the first slit a repeated tangential deformation of the first sidewall section so as to narrow the first slit is effectively prevented. Consequently, a disengagement of the latch element and the first through recess is effectively inhibited and prevented. In this way, a long-lasting, durable and non-releasable connection of cartridge holder and body is provided.

According to a further embodiment the ramp section is located at or near a proximal end of the receptacle. It may be located and positioned at or near a proximal end of the first protrusion. In this way it will be only a proximal end section of the insert section of the cartridge holder that will become subject to a temporary radially inwardly directed deflection. In this way the total degree of a radially inwardly directed elastic deformation of the cartridge holder can be limited to a proximal end section thereof. Moreover, and since only a lateral edge of the first sidewall section of the insert section engages with the ramp section and its beveled surface it is only a small portion of the first sidewall section that is subject to a radial deflection. Having a radial deflection of the insert section only at a proximal end leaves any further sections of the insert section of the sidewall of the cartridge holder rather unaffected in terms of a radial deformation.

The area of the sidewall of the insert section that is subject to a radial deformation can be minimized, which is beneficial for reducing eventual mechanical load acting in radial direction onto the cartridge located inside the cartridge holder. Moreover, it is conceivable, that the proximal end of the insert section of the cartridge holder which is subject to a temporary radially inwardly directed deflection is located proximally offset from a proximal end of the cartridge. In this way a radially inwardly directed mechanical load to the cartridge can be completely avoided during the assembly process of body and cartridge holder.

Furthermore and according to another embodiment the lateral abutment face of the first protrusion adjoins the ramp section. The lateral abutment face typically faces in tangential direction. It may extend perpendicular to the beveled surface of the ramp section. The beveled surface typically faces in distal direction as well as radially inwardly. The lateral abutment face typically extends in radial and longitudinal direction. The surface normal of the lateral abutment face typically faces towards the tangential direction so as to engage with the correspondingly-shaped lateral abutment face of the first sidewall section of the insert section confining the first slit.

References made to the axial or radial as well as to a tangential direction typically refer to a substantially tubular or cylindrical geometry of the body and of the cartridge holder. However, these expressions equally relate to other elongated geometries of body and cartridge holder. If for instance the body and the cartridge holder, in particular the receptacle of the body and the insert section of the cartridge holder comprise a somewhat oval cross-section, terms like 'axial', 'tangential' and 'radial' are to be understood as if the insert section and the receptacle were of cylindrical shape. Hence the axial direction coincides with the main longitudinal axis of body or cartridge holder. The radial direction points from a center axis outwardly and perpendicular to the elongation of cartridge holder and body and the tangential direction or lateral is perpendicular to the radial direction and the longitudinal direction.

Furthermore, the above described positive engagement and interconnection of body and cartridge holder principally works with a single first through recess engaging with a single first latch element, it may be of particular benefit to have a second through recess as well as a second latch element. Typically, the cartridge holder may comprise a first through recess and a second through recess at a oppositely located sidewall section, e.g. at diametrically opposite sidewall sections.

Correspondingly, also the body may comprise a first latch element to engage with the first through recess and a second latch element to engage with the second through recess. Likewise, the cartridge holder may also comprise not only a single first slit but a first and a second slit that may be also located opposite to each other on or in the sidewall of the insert section.

Correspondingly, also the body may comprise not only a single first protrusion but may comprise a first protrusion and a second protrusion, wherein the first and the second protrusions are located opposite to each other on the inside of the sidewall of the receptacle. By increasing the number of mutually engaging through recesses, latch elements, slits and protrusions the stability and durability of the interconnection of cartridge holder and body can be further improved.

Typically, and as seen in tangential direction through recesses and slits are arranged in an alternating way at the sidewall of the insert section. Correspondingly, the latch elements and protrusions located on the inside of the sidewall of the receptacle are arranged alternately as seen in tangential direction. An alternating and eventually symmetrical arrangement of mutually engaging latch elements, through recesses, protrusions and slits further improves the durability and stability of the interconnection of the body and the cartridge holder.

According to another aspect there is also provided an injection device for administering an injectable medicament into biological tissue. The injection device, which is typically configured as a handheld injection device such as an injection pen comprises a housing as described above. The injection device further comprises a drive mechanism assembled inside the body of the housing and further comprises a cartridge that is assembled inside the cartridge holder. Typically, the injection device is configured as a pre-filled injection device or as a disposable injection device with the cartridge radially assembled therein. The injection device may be configured for a one-time use or for several consecutive usages.

After the content of the cartridge has been used up the entire device may be intended to be discarded in its entirety. Hence, the drive mechanism is not configured for a reset operation nor is the cartridge intended to be replaced by a new one. When configured and designed as a disposable or prefilled injection device the positively engaging first and second latch elements of the body and of the cartridge holder are configured to be of permanent and non-releasable type.

Once the first and second latch elements positively engage there is no way of disengaging the same except when destroying one of the housing components' body or cartridge holder.

In the present context, the distal direction points in the direction of the dispensing and of the device, where, preferably a needle assembly is provided having a double-tipped injection needle that is to be inserted into biological tissue or into the skin of a patient for delivery of the medicament.

The proximal end or proximal direction denotes the end of the device or a component thereof, which is furthest away from the dispensing end. Typically, an actuating member is located at the proximal end of the injection device, which is directly operable by a user to be rotated for setting of a dose and which is operable to be depressed in distal direction for dispensing of a dose.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (~150kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

It will be further apparent to those skilled in the art that various modifications and variations can be made to the present invention without departing from the spirit and scope of the invention. Further, it is to be noted, that any reference numerals used in the appended claims are not to be construed as limiting the scope of the invention.

### Brief description of the drawings

In the following, a non-limiting embodiment of the invention is described in detail by making reference to the drawings, in which:
- Fig. 1: shows a perspective and partially cut view of the distal end of a body of a housing of an injection device,
- Fig. 2: shows a perspective view of a cartridge holder to be connected to the body according to Fig. 1,
- Fig. 3: is an enlarged perspective view of the insert section of the cartridge holder,
- Fig. 4: is an enlarged and partially cut view of the cartridge holder and of the body at the beginning of an insertion of the cartridge holder's insert section into the receptacle of the body,
- Fig. 5: is a view according to Fig. 4 but with cartridge holder and body in a final assembly configuration,
- Fig. 6: is a view according to Figs. 4 and 5 with the assembly of body and cartridge holder rotated by 90 degrees with respect to the longitudinal axis,
- Fig. 7: is an enlarged and partial view of a slit in the sidewall of the insert section of the cartridge holder in an initial unbiased state,
- Fig. 8: is the same view as Fig. 7 but with the sidewall section of the cartridge holder in a biased state in which the first slit is narrowed due to a tangentially directed deflection of the sidewall section,
- Fig. 9: is a partially cut and perspective view during insertion of the insert section of the cartridge holder into the receptacle prior to or at the beginning of a tangential deflection of a sidewall section of the cartridge holder's insert section,
- Fig. 10: is the same view as Fig. 9 but with cartridge holder and body during a tangential deflection and deformation of a sidewall section of the insert section of the cartridge holder prior to reach a final state of assembly,
- Fig. 11: is a perspective view according to Figs. 9 and 10 when the cartridge holder and the body have reached the final assembly configuration,
- Fig. 12: is an exploded view of an exemplary injection device equipped with a housing in accordance to Figs. 1-11 and
- Fig. 13: is a longitudinal cross-section through the injection device according to Fig. 12.

### Detailed description

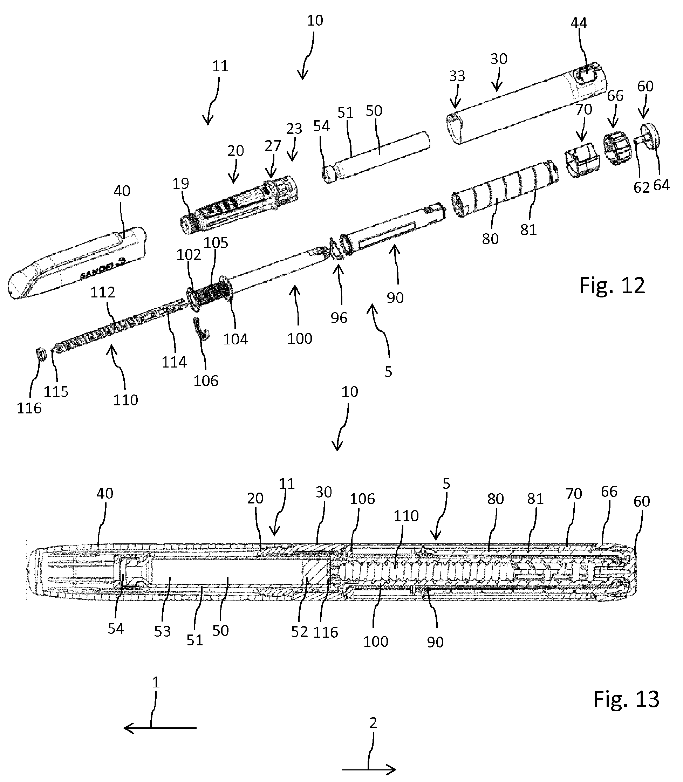

The injection device 10 as shown in Figs 12 and 13 is configured as a pen-type injector. It comprises an elongated housing 11 extending in a longitudinal or axial direction. Towards a distal direction 1 the injection device 10 comprises a distal housing component denoted as cartridge holder 20. In the opposite longitudinal direction the housing 11 comprises a second housing component denoted as body 30. Both housing components, namely cartridge holder 20 and body 30 are of tubular and elongated shape. The cartridge holder 20 is configured to accommodate a cartridge 50 comprising a tubular-shaped barrel 51 filled with a liquid medicament 53. At a distal end the cartridge 50 comprises a pierceable seal 54 typically inform of a pierceable septum of an elastomeric material.

At the opposite proximal end the cartridge 50 is sealed by a piston 52 slidably arranged inside the barrel 51 of the cartridge 50. For dispensing of a dose of the liquid medicament 53 the cartridge holder 20 comprises a threaded socket 19 at its distal end to receive a correspondingly threaded needle assembly with a double-tipped injection needle. A proximal tipped end of the injection needle of the needle assembly, which is presently not illustrated, is configured to pierce the distal seal 54 of the cartridge 50 thereby gaining access to the interior of the cartridge 50. The distal end of the injection needle is then configured to puncture biological tissue to deliver the medicament into biological tissue. For medicament delivery the piston 52 is to be displaced in distal direction 1 under the action of a distally advancing piston rod 110 of a drive mechanism 5 of the injection device 10. The drive mechanism 5 is accommodated and fixed in the body 30 of the injection device 10. There is further provided a protective cap 40 covering the distal portion of the cartridge holder 20 when assembled and attached thereto.

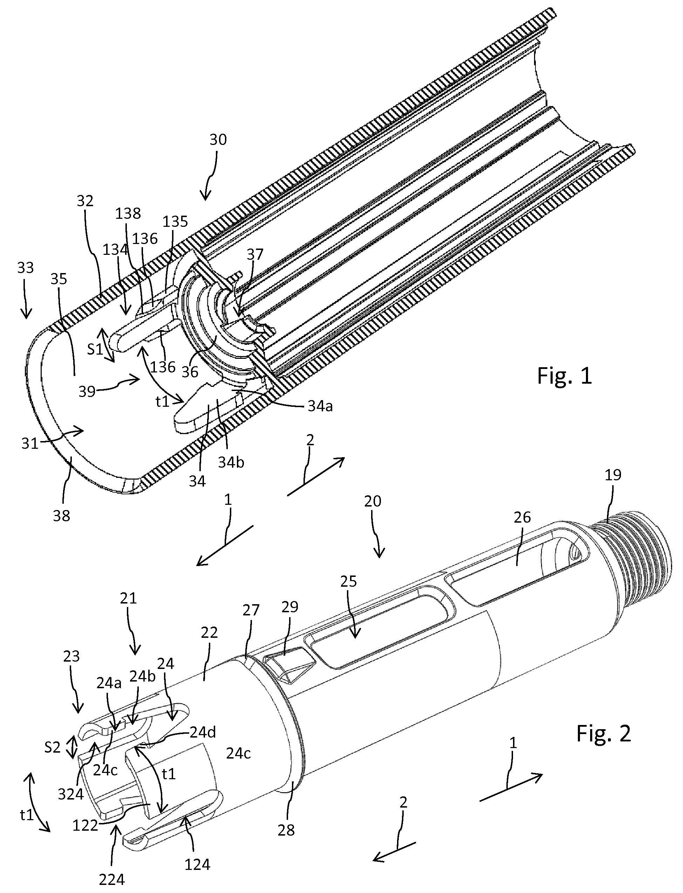

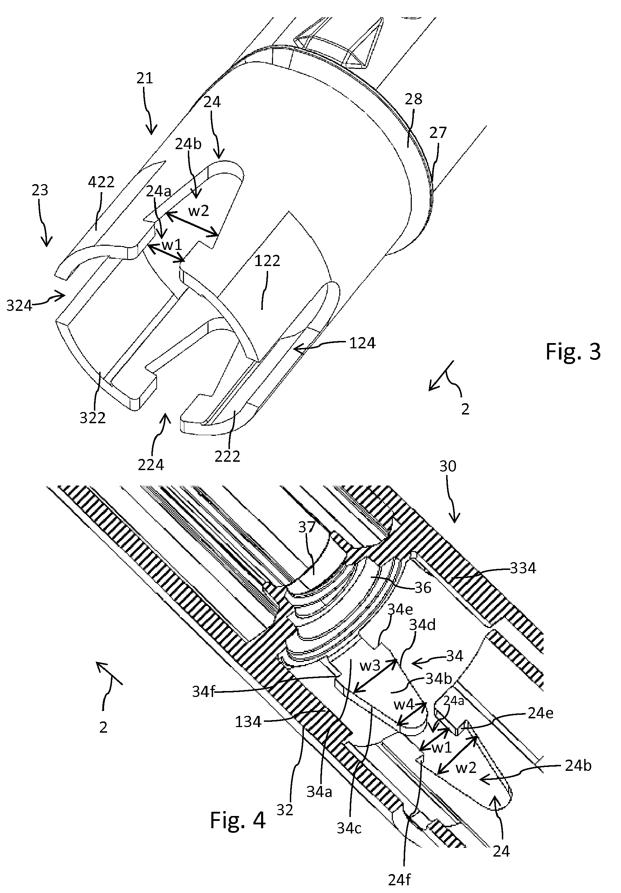

The cartridge holder 20 and the body 30 are to be interconnected by means of a positive connection as it is explicitly shown in Fig. 4. As illustrated in Figs 1 and 2 the cartridge holder 20 comprises a proximal end 23 to interconnect non-releasable with a distal end 33 of the body 30. The cartridge holder 20 and the body 30 are interconnectable in an interleaved or nested way. In the presently illustrated embodiment the proximal connecting end of the cartridge holder 20 comprises an insert section 21 axially confined in distal direction 1 by a radially outwardly extending annular ridge or flange section 27. The distal connecting end of the body 30 comprises a receptacle 31 to axially receive the insert section 21 of the cartridge holder 20. The outer diameter of the insert section 21 exactly matches with the inner diameter of the receptacle 31 so that the insert section 21 can be inserted into the receptacle 31 by means of a sliding motion in proximal direction 2 relative to the body 30.

From the combination of the Figs. 2-4 it is immediately apparent, that the insert section 21 of the cartridge holder 20 is located at a proximal end 23 of the cartridge holder 20 and that the receptacle 31 is located at a distal end 33 of the body 30. Once mutually assembled the insert section 21 of the cartridge holder 20 is fully or entirely located inside the receptacle 31 of the body 30.

The annular flange section 27 radially outwardly protruding from the sidewall 22 of the insert section 21 comprises an abutment face 28 facing in proximal direction 2. When assembled with the body 30 the abutment face 28 is in axial abutment with a distal end face 38 of the sidewall 32 of the body 30. Additionally and as it is apparent from Fig. 4 the proximal end 23 of the cartridge holder 20 may optionally also axially abut with a web 36 extending across the inner diameter of the tubular-shaped body 30. The web 36 comprises a central through opening 37 that may be threaded. The threaded through opening 37 may be threadedly engaged with the piston rod 110, in particular with a distal thread 112 of the piston rod 110 of the drive mechanism 5 as illustrated in Figs. 12 and 13. The web 36 may comprise a closed surface and confines the receptacle 31 of the body 30 in proximal direction.

As further illustrated in Fig. 2 the cartridge holder 20 comprises a cartridge receiving space 25 in its interior that is typically completely occupied by the cartridge 50. Near a distal end or in an intermediate section the sidewall 22 of the cartridge holder 20 further comprises at least one transparent window 26 or a respective through recess in order to provide visual inspection of the cartridge 50 located therein. There is further shown a radially outwardly extending protrusion 29 in close proximity to the flange section 27. The protrusion 29 which is located distally from the flange section 27 and which is hence visible from outside when the injection device 10 is configured to engage with a correspondingly-shaped protrusion or snap feature on the inside of the sidewall of the protective cap 40. In this way a snap-fit or positive engagement of cartridge holder 20 and protective cap 40 can be provided.

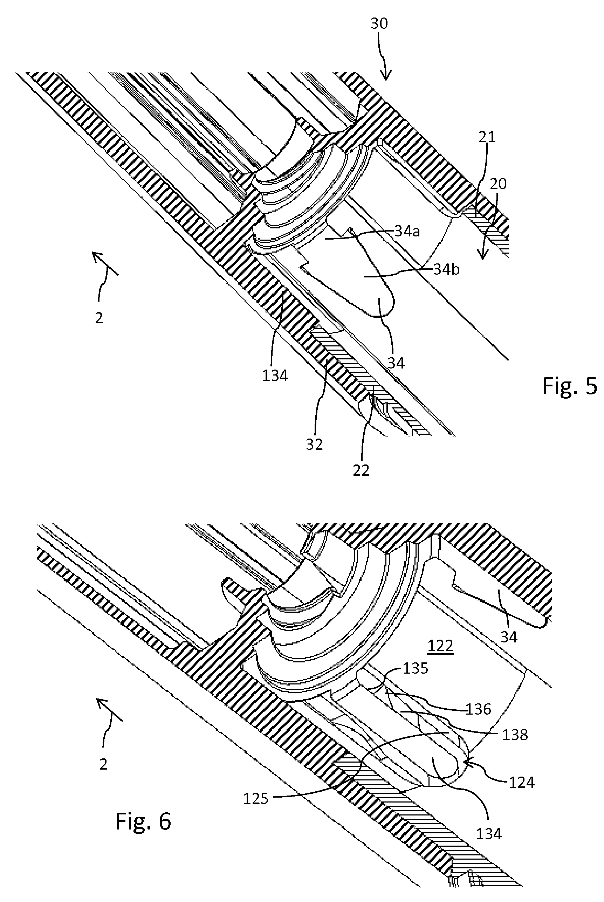

As it is apparent from Figs. 1 and 2 as well as from Figs. 4 and 5 there is provided at least a first through recess 24 in the sidewall 22 of the insert section 21 of the cartridge holder 20. The first through recess comprises a neck receiving portion 24a having a first width w1. The neck receiving portion 24a axially extends into a laterally or tangentially widened latch receiving portion 24b of the first through recess 24. The latch receiving portion 24b comprises a second width w2 that is larger than the first width w1.

As further shown in Figs. 2 and 3 the first through recess 24 adjoins the proximal end 23 of the cartridge holder 20. The first through recess 24 is hence open towards the proximal direction 2. It is configured to receive a latch element 34 protruding radially inwardly from the inside 35 of the sidewall 32 of the receptacle 31.

The radial extension or radial protrusion of the first latch element 34 substantially matches with the thickness of the sidewall 22 of the insert section 21 of the cartridge holder 20. So when fully assembled as illustrated in Fig. 5 the first latch element 34 entirely occupying the first through recess 24 forms a somewhat even and closed inner surface with the inside of the sidewall 22 of the insert section 21.

The latch element 34 comprises a latch portion 34b and an adjoining neck portion 34a. The neck portion 34a extends in axial distal direction into the latch portion 34b. As illustrated in Figs. 1, 4 and 5 the latch portion 34b is wedge-shaped and comprises an increasing tangential width in proximal direction 2. Hence, the latch portion 34b comprises a minimal width w4 at a distal end as shown in Fig. 4. Near a proximal end and close or in direct vicinity to the neck portion 34a the latch portion 34b comprises a maximal width w3 in tangential direction. The maximal width w3 matches with the second width w2 of the latch receiving portion 24b of the first through recess 24.

As it is exemplary illustrated in Fig. 4, the minimal width w4 at the distal end of the latch element 34b is smaller than the first width w1 of the neck receiving portion 24a of the first through recess 24. So for inserting the first latch element 34 into the first through recess 24 along the axial longitudinal direction, and in particular in distal direction 1 the distal end of the wedge-shaped first latch element 34 will easily enter the narrowed neck receiving portion 24a of the first through recess 24. Upon a further distally directed displacement of the body 30 relative to the cartridge holder 20 the side faces 34c and 34d of the latch element 34 will engage with correspondingly-shaped side faces 24c of the insert section that laterally or tangentially confine the neck receiving portion 24a.

Since the latch portion 34b continuously and smoothly widens in tangential direction towards the proximal direction and since the latch portion 34b is urged further into the neck receiving portion 24a the side faces 34c, 34d of the latch portion 34b will start to abut and to engage with the side faces 24c, 24d of the insert section 21. Consequently and as the maximal width w3 of the latch portion 34b is larger than the first width w1 and since the first latch element 34, in particular its latch portion 34b is substantially inelastic the insertion of the latch portion 34b into the first through recess 24 leads to a temporary deformation of the insert section 21 of the cartridge holder 20. Consequently, the neck receiving portion 24a of the first through recess 24 will be subject to a widening in tangential or lateral direction.

As a consequence a first sidewall section 122 of the insert section 21 that tangentially confines the first through recess 24 will be subject to a lateral or tangential deformation so as to give way for the widened latch portion 34 of the first latch element. When reaching a final assembly configuration in which the latch element 34 is completely received inside the first through recess 24 abutment faces 34e and 34f at the proximal end of the latch portion 34b will engage with correspondingly-shaped abutment faces 24e and 24f that are located at the proximal end of the latch receiving portion 24b of the first through recess 24. The abutment faces 34e and 34f face in proximal direction 2 and extend in tangential direction inwardly from the proximal end of the latch portion 34b. Via the abutment faces 34e and 34f the latch portion 34b extends into the tangentially narrowed neck portion 34a.

The correspondingly-shaped abutment faces 24e and 24f of the first through recess 24 face in distal direction 1 so as to axially abut with the abutment faces 34e and 34f. Once a final assembly configuration has been reached as shown in Fig. 5, the abutment faces 24e and 34e as well as the abutment faces 24f and 34f are in axial abutment so as to prevent a sliding movement of the cartridge holder 20 in distal direction relative to the body 30. In this way an axial interlock of cartridge holder 20 and body 30 is obtained. The mutual engagement of the first through recess 24 with the first latch element 34 provides an axial interlock that inhibits a separation of cartridge holder 20 and body 30 in axial direction. The engagement of the end face 38 with the abutment 28 of the annular flange section 27 as well as an eventual axial abutment of the proximal end 23 of the cartridge holder 20 with the web 36 extending across the inner cross-section of the body 30 prevent a further insertion of the insert section 20 into the body 30. This abutment therefore provides a kind of an axial interlock that prevents a further movement of the cartridge holder 20 in proximal direction relative to the body 30.

Since the first through recess 24 is open towards the proximal direction 2 the positive engagement of the first through recess 24 with the first latch element 34 can be obtained without any radial deformation of the insert section 21 of the cartridge holder. In this way the cartridge 50 already assembled inside the cartridge holder 20 prior to the final assembly step of interconnecting body 30 and cartridge holder 20 will not be subject to mechanical load acting in radial direction. In this way mechanical stress eventually applied to the cartridge during the final step of assembly of body 30 and cartridge holder 20 can be effectively reduced. A likelihood of cartridge breakage can be also reduced which enhances production quality and patient safety.

From Fig. 2 as well as from Fig. 6 it is immediately apparent that the cartridge holder comprises not only the first through recess 24 but also a second through recess 224. In the present embodiment the second through recess 224 is located diametrically opposite to the first through recess 24. Correspondingly also the body 30 comprises not only a first latch element 34 but also a second latch element which is not shown explicitly in the Figures but which is identically shaped and configured as the first latch element 34. The second latch element is also located opposite to the first latch element 34. By having two pairs of through recesses and latch elements, a twofold positive interconnection of body 30 and cartridge holder 20 can be provided thus improving the stability and durability of the positive interconnection of body 30 and cartridge holder 20.

In addition to the mutual engagement of at least the first through recess 24 with the first latch element 34 the insert section 21 of the cartridge holder 20 further comprises a first slit 124 that adjoins the proximal end 23 and which extends in distal direction 1 from the proximal end 23. The first slit 124 is located at a first tangential or lateral distance t1 from the first through recess 24 as illustrated in Fig. 2. Correspondingly to the first slit 124 also the receptacle 31 comprises a first protrusion 134 that is axially elongated and which extends inwardly from the inside 35 of the sidewall 32 of the receptacle 31. The first protrusion 134 is located at the first tangential or lateral distance t1 from the first latch element 34.

A transverse size S1 of the first protrusion 134 is substantially equal to a transverse size S2 of the second through recess 124 when the first sidewall section 122 is in an unbiased state. At least an axial section of the first protrusion, typically a proximal end section of the first protrusion 134 comprises a transverse size S1, hence an expansion in tangential direction that is substantially identical to the transverse size S2 of the first slit 124. Upon final assembly of body 30 and cartridge holder 20 not only the first latch element 34 engages with the first through recess 24 but also the first protrusion 134 axially enters the first slit 124. The first slit 124 and the first protrusion 134 may engage simultaneously with the engagement of the first latch element 34 and the first through recess 24. The engagement of the first protrusion 134 with the first slit 124 provides an additional axial and longitudinal guiding for the insert section 21 sliding into the receptacle 31 in proximal direction 2 relative to the body 30.

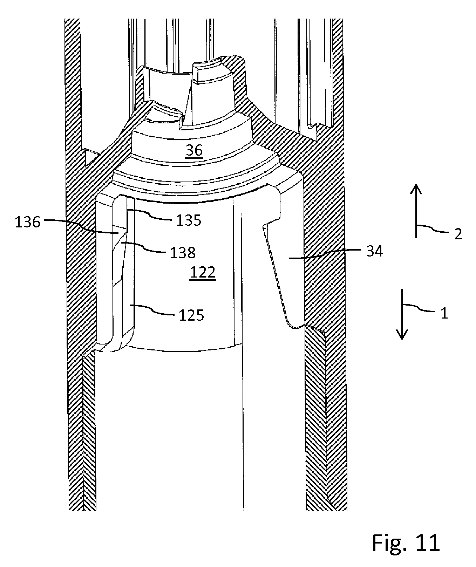

Apart from that and when reaching a final assembly configuration as shown in Figs. 6 and 11 a lateral abutment face 125 of a first sidewall section 122 of the insert section 21 that confines the first slit 124 is in tight tangential or circumferential engagement with a lateral abutment face 135 of the first protrusion 134. As shown in Fig. 11 and when a final assembly configuration has been reached the sidewall section 122 located laterally or tangentially between the first slit 124 and the first through recess 24 is tangentially sandwiched between the first latch element 34 and the first protrusion 134. Hence, when fully assembled the first sidewall section 122 of the insert section 21 of the cartridge holder 20 entirely occupies a free space 39 laterally or tangentially between the first latch element 34 and the first protrusion 134. The lateral abutment of abutment faces 125 and 135 actually prevent a deformation of the first sidewall section 122 towards the tangential or lateral direction.

This serves to prevent a release or disengagement of the first latch element 34 and the first through recess 24. Once the positive interconnection of body 30 and housing 20 is attained it cannot be abrogated without destroying at least one of the housing component's cartridge holder 20 or body 30.

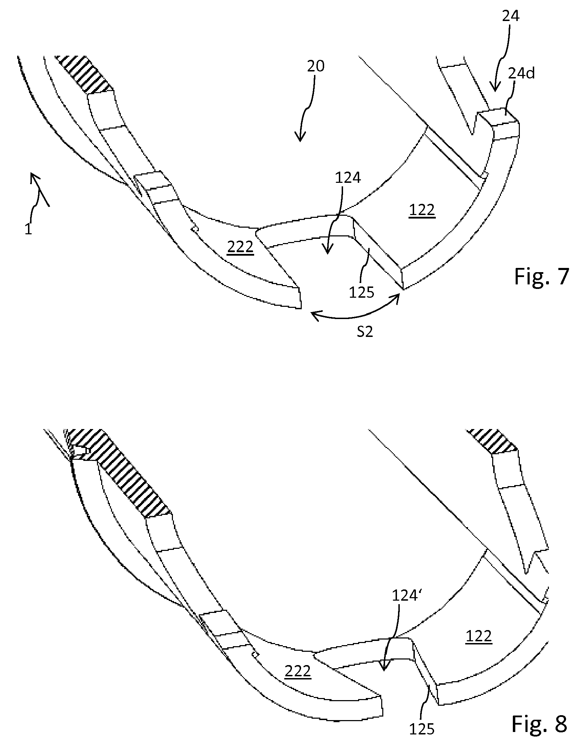

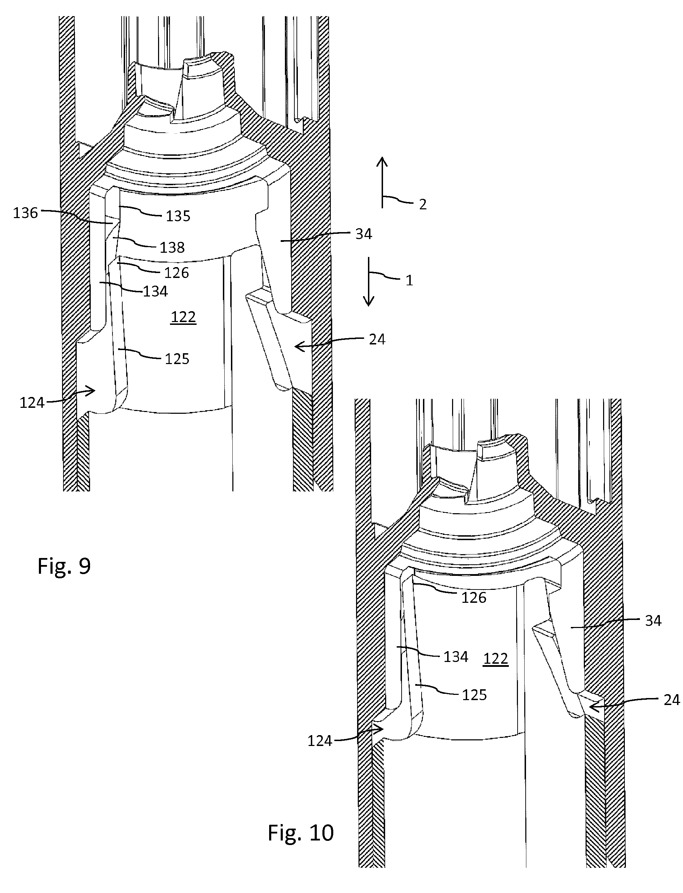

Since the geometric shape, especially the tangential width of the first sidewall section 122 exactly matches with the free space 39 between the first latch element 34 and the first protrusion 134 it would be quite difficult to arrive at the final assembly configuration as shown in Fig. 11 since the first sidewall section 122 is subject to a tangentially directed elastic deformation during the process of assembly as described above. In Figs. 7 and 8 the degree of tangential deformation of the first sidewall section 122 is apparent. In Fig. 7 the initial unbiased state is illustrated that corresponds to the geometry of the insert section 21 according to Fig. 4. In Fig. 8 a biased state of the first sidewall section 122 is shown that substantially corresponds to the configuration of Fig. 10, in which a final assembly configuration has been almost reached.

Since the neck receiving portion 24a of the first through recess 24 is subject to a tangential widening, the first sidewall section 122 is subject to a respective tangential deformation at the expense of the tangential width of the first slit 124'. From a comparison of Figs. 7 and 8 it is immediately apparent that the first slit 124' in Fig. 8 is narrower in tangential or circumferential direction compared to the first slit 124 with the first sidewall section 122 being in the unbiased initial state.

Since the tangential width of the first slit 124 exactly matches with the tangential width of the first protrusion 134 the first protrusion 134 will be unable to completely enter the first slit 124 as long as the first sidewall section 122 is subject to an elastic deformation. For enabling and facilitating the process of assembly, i.e. the sliding motion of the insert section 21 into the receptacle 31, the first protrusion 134 comprises a ramp section 136 as best seen in Fig. 1. Typically, the longitudinally extending first protrusion 134 comprises two ramp sections 136 near its proximal end, hence near or adjoining the web 36. The at least one ramp section 136 comprises a beveled surface 138 facing in distal direction 1. The beveled surface 138 extends from the inside 35 of the sidewall 32 of the receptacle radially inwardly as seen in proximal direction 2.

Hence, as seen from the distal end towards the proximal end of the first protrusion the ramp section 136 comprises a gradually and continuously increasing extension radially inwardly from the inside 35 of the sidewall 32. The distal end of the ramp section 136 is integrally formed with the inside 35 of the sidewall 32. The distal end of the ramp section 136 is flush with the inside 35 of the sidewall 32. As the insert section 21 of the cartridge holder 20 is pushed into the receptacle 31 as shown in the sequence of Figs. 9-11 a proximal and lateral edge 126 of the first sidewall section 122 engages with the ramp section 136 of the first protrusion 134. In this way the proximal and lateral edge 126 of the first sidewall section 122 is subject to a small but distinct radially inwardly directed deformation and deflection. Prior to reach a final state of assembly the lateral edge 126 of the first sidewall section 122 radially overlaps with at least the ramp section 136 of the first protrusion 134. This radial overlap persists during the process of assembly and as long as the first sidewall section is subject to a tangential deflection from its initial unbiased state.

Due to the radial deformation of the edge 126 the first sidewall section 122 is allowed to flex and to deform in tangential or lateral direction towards the first protrusion 134 and to radially overlap at least in sections with the first protrusion 134. When the final assembly configuration has been reached as shown in Fig. 11 the first sidewall section 122 returns into its unbiased state thereby increasing the lateral or tangential size of the first slit 124 to the initial size. Consequently and due to the mutually matching geometries of the first sidewall section 122 and the free space 39 between the first latch element 34 and the first protrusion 134 the first sidewall section, in particular the proximal and lateral edge 126 thereof is free to relax radially outwardly so as to enter and to occupy the free space 39.

As it is shown in Figs. 1 and 11 the ramp portion 136 with its beveled surface 138 is located at a proximal end of the receptacle 31. It is hence operable to deflect only a limited proximal and lateral edge 126 of the insert section 21 of the cartridge holder 20. Positioning of the ramp section 136 towards a proximal end of the interface of insert section 21 and receptacle 31 is beneficial to reduce any radially inwardly directed mechanical load that may otherwise be present to the cartridge 50. If the radially inwardly directed deflection of the proximal and lateral edge 126 of the first sidewall section 122 should be of such a magnitude that the inside of the first sidewall section 122 gets in mechanical contact with the barrel 51 of the cartridge 50 the mechanical load will be present to a proximal end of the cartridge, which is stabilized by the piston 54 as well as by an enamel edge of the vitreous body 51 of the cartridge.

In the present embodiment there are provided two protrusions, 134 and 334, that are provided on opposite, e.g. geometrically opposite portions on the inside 35 of the sidewall 32 of the receptacle 31. Likewise and corresponding to the position and shape of the first protrusion 134 and the second protrusion 334 there are provided a first slit 124 to receive the first protrusion 134 as well as a second slit 324 to receive the second protrusion 334.

In the illustrated embodiment the first and the second protrusions 134 and 334 are located diametrically opposite. Correspondingly, also the first slit 124 and the second slit 324 are located diametrically opposite on the insert section 21. Logically, there is not only a first sidewall section 122 as described above, but there exists altogether four sidewall sections 122, 222, 322 and 422 that are simultaneously subject to a tangential deformation as the first and second latch elements 34 engage with correspondingly-shaped first and second through recesses 24, 224. In this context it is further to be mentioned, that the present concept of establishing a snap-fit connection of cartridge holder 20 and body 30 is not limited to only two pairs of through recesses and latch elements or two pairs of slits and protrusions but may be expanded to an arbitrary number of mutually positively engaging structures.

In the following the general functionality and features of the drive mechanism 5 is described. The drive mechanism 5 is only exemplary for one of a plurality of differently configured drive mechanisms that are generally implementable in a disposable pen-injector. The drive mechanism as described above is explained in more detail e.g. in WO2004/078239A1, WO 2004/078240A1 or WO 2004/078241 A1 the entirety of which being incorporated herein by reference. The interface and interconnection of housing components, such like the cartridge holder 20 and the body 30 as explained above can be generally implemented with a large variety of different drive mechanisms 5 and injection devices.

The drive mechanism 5 comprises numerous mechanically interacting components. A flange like web 36 or support of the body 30 comprises a threaded axial through opening 37 threadedly engaged with a distal thread 112 of the piston rod 110. The distal end of the piston rod 110 comprises a bearing 115 on which a pressure foot 116 is free to rotate with the longitudinal axis of the piston rod 110 as an axis of rotation. The pressure foot 116 is configured to axially abut against the proximally facing thrust receiving surface of the piston 52 of the cartridge 50. During a dispensing action the piston rod 110 rotates relative to the body 30 thereby experiencing a distally directed advancing motion relative to the body 30 and hence relative to the barrel 51 of the cartridge 50. As a consequence, the piston 52 of the cartridge 50 is displaced in distal direction by a well-defined distance due to the threaded engagement of the piston rod 110 with the body 30.

The piston rod 110 is further provided with a second thread 114 at its proximal end. The distal thread 112 and the proximal thread 114 are oppositely handed.

There is further provided a drive sleeve 100 having a hollow interior to receive the piston rod 110. The drive sleeve 100 comprises an inner thread threadedly engaged with the proximal thread 114 of the piston rod 110. Moreover, the drive sleeve 100 comprises an outer threaded section 105 at its distal end. The threaded section is axially confined between a distal flange portion 102 and another flange portion 104 located at a predefined axial distance from the distal flange portion 102. Between the two flange portions 102, 104 there is provided a last dose limiting member 106 in form of a semi-circular nut having an internal thread matching the threaded section 105 of the drive sleeve 100.

The last dose limiting member 106 further comprises a radial recess or protrusion at its outer circumference to engage with a complementary-shaped recess or protrusion at an inside of the sidewall 32 of the body 30. In this way the last dose limiting member 106 is splined to the body 30. A rotation of the drive sleeve 100 in a dose incrementing or clockwise direction during consecutive dose setting procedures leads to an accumulative axial displacement of the last dose limiting member 106 relative to the drive sleeve 100. There is further provided an annular spring 96 that is in axial abutment with a proximally facing surface of the flange portion 104. Moreover, there is provided a tubular-shaped clutch member 90. At a first end the clutch member 90 is provided with a series of circumferentially directed saw teeth. Towards a second opposite end of the clutch member 90 there is located a radially inwardly directed flange.

Furthermore, there is provided a dose dial or dose indicating sleeve 80. The dose indicating sleeve 80 is provided outside of the spring 96 and the clutch member 90 and is located radially inward of the body 30. A helical groove 81 is provided about an outer surface of the dose indicating sleeve 80. The body 30 is provided with a window 44 through which a part of the outer surface of the dose indicating sleeve 80 can be seen. The body 30 is further provided with a helical rib at an inside sidewall portion of an insert piece 70, which helical rib is to be seated in the helical groove 81 of the dose indicating sleeve 80. The tubular shaped insert piece 70 is inserted into the proximal end of the body 30. It is rotationally and axially fixed to the body 30. There are provided first and second stops on the body 30 to limit a dose setting procedure during which the dose indicating sleeve 80 is rotated in a helical motion relative to the body 30.

A dose dial grip 66 is disposed about an outer surface of the proximal end of the dose indicating sleeve 80. An outer diameter of the dose dial 66 typically corresponds to the outer diameter of the body 30. The dose dial 66 is secured to the dose indicating sleeve 80 to prevent relative movement there between. The dose dial 66 is provided with a central opening.

Furthermore, a dose button 60 of generally T-shape is provided at a proximal end of the injection device 10. A stem 62 of the dose button 60 extends through the opening in the dose dial 66 through an inner diameter of extensions of the drive sleeve 100 and into a receiving recess at the proximal end of the piston rod 110. The stem 62 is retained for limited axial movement in the drive sleeve 100 and against rotation with respect thereto. A head 64 of the dose button 60 is generally circular. A skirt extends from a periphery of the head 64 and is further adapted to be seated in a proximally accessible annular recess of the dose dial 66.

To dial a dose a user rotates the dose dial 66. With the spring 96 also acting as a clicker and the clutch member 90 engaged, the drive sleeve 100 the spring or clicker 96, the clutch member 90 and the dose indicating sleeve 80 rotate with the dose dial 66. Audible and tactile feedback of the dose being dialed is provided by the spring 96 and by the clutch member 90. Torque is transmitted through saw teeth between the spring 96 and the clutch member 90. The helical groove 81 on the dose indicating sleeve 80 and a helical groove in the drive sleeve 100 have the same lead. This allows the dose indicating sleeve 80 to extend from the body 30 and the drive sleeve 100 to climb the piston rod 110 at the same rate. At a limit of travel a radial stop on the dose indicating sleeve 80 engages either with a first stop or a second stop provided on the body 30 to prevent further movement. Rotation of the piston rod 110 is prevented due to the opposing directions of the overall and driven threads on the piston rod 110.

The last dose limiting member 106 keyed to the body 30 is advanced along the threaded section 105 by the rotation of the drive sleeve 100. When a final dose dispensed position is reached, a radial stop formed on a surface of the last dose limiting member 106 abuts a radial stop on the flange portion 104 of the drive sleeve 100, preventing both, the last dose limiting member 106 and the drive sleeve 100 from rotating further.

Should a user inadvertently dial beyond the desired dosage, the pen-injector 10 allows the dosage to be dialed down without dispense of the medicament from the cartridge 50. For this the dose dial 66 is simply counter-rotated. This causes the system to act in reverse. A flexible arm of the spring or clicker 96 then acts as a ratchet preventing the spring 96 from rotating. The torque transmitted through the clutch member 90 causes the saw teeth to ride over one another to create the clicks corresponding to dialed dose reduction. Typically, the saw teeth are so disposed that a circumferential extent of each saw tooth corresponds to a unit dose.

When the desired dose has been dialed the user may simply dispense the set dose by depressing the dose button 60. This displaces the clutch member 90 axially with respect to the dose indicating sleeve 80 causing dog teeth thereof to disengage. However, the clutch member 90 remains keyed in rotation to the drive sleeve 100. The dose indicating sleeve 80 and the dose dial 66 are now free to rotate in accordance with the helical groove 81.

The axial movement deforms the flexible arm of the spring 96 to ensure the saw teeth cannot be overhauled during dispense. This prevents the drive sleeve 100 from rotating with respect to the body 30 though it is still free to move axially with respect thereto. The deformation is subsequently used to urge the spring 96 and the clutch member 90 back along the drive sleeve 100 to restore the connection between the clutch member 90 and the dose indicating sleeve 80 when the distally directed dispensing pressure is removed from the dose button 60.

The longitudinal axial movement of the drive sleeve 100 causes the piston rod 110 to rotate through the through opening 133 of the support 132 of the body 30, thereby to advance the piston 52 in the cartridge 50. Once the dialed dose has been dispensed, the dose indicating sleeve 80 is prevented from further rotation by contact of a plurality of members extending from the dose dial 66 with a corresponding plurality of stops. A zero dose position is finally determined by the abutment of one of axially extending edges of members of the dose indicating sleeve 80 with a corresponding stop of the body 30.

### Reference numbers

- 1: distal direction
- 2: proximal direction
- 5: drive mechanism
- 10: injection device
- 11: housing
- 19: threaded socket
- 20: cartridge holder
- 21: insert section
- 22: sidewall
- 23: proximal end
- 24: through recess
- 24a: neck receiving portion
- 24b: latch receiving portion
- 24c: side face
- 24d: side face
- 24e: abutment face
- 24f: abutment face
- 25: cartridge receiving space
- 26: windos
- 27: flange section
- 28: abutment face
- 29: protrusion
- 30: body
- 31: receptacle
- 32: sidewall
- 33: distal end
- 34: latch element
- 34a: neck portion
- 34b: latch portion
- 34c: side face
- 34d: side face
- 34e: abutment face
- 34f: abutment face
- 35: inside
- 36: web
- 37: through opening
- 38: end face
- 39: free space
- 40: cap
- 44: window
- 50: cartridge
- 51: barrel
- 52: piston
- 53: medicament
- 54: seal
- 60: dose button
- 62: stem
- 64: head
- 66: dose dial
- 70: insert piece
- 80: dose indicating sleeve
- 81: helical groove
- 90: clutch member
- 96: spring
- 100: drive sleeve
- 102: distal flange portion
- 104: flange portion
- 105: threaded section
- 106: last dose limiting member
- 110: piston rod
- 112: distal thread
- 114: proximal thread
- 115: bearing
- 116: pressure foot
- 122: sidewall section
- 124: slit
- 125: abutment face
- 126: edge
- 134: protrusion
- 135: abutment face
- 136: ramp section
- 138: beveled surface
- 222: sidewall section
- 224: through recess
- 322: sidewall section
- 324: slit
- 334: protrusion
- 422: sidewall section

## Claims

1. A housing for an injection device, the housing comprising:
- a body (30) to accommodate a drive mechanism (5) to operably engage with a piston (52) of a cartridge (50) filled with a liquid injectable medicament (53),
- a cartridge holder (20) having a cartridge receiving space (25) to accommodate the cartridge (50),
- wherein the cartridge holder (20) and the body (30) comprise an elongated shape and extend along an axial longitudinal direction (1, 2),
- wherein the cartridge holder (20) has an insert section (21) at a proximal end (23),
- wherein the body (30) has a receptacle (31) at a distal end (33) to receive the insert section (21) of the cartridge holder (20),
- wherein the body (30) comprises a first latch element (34) on an inside (35) of a sidewall (32) of the receptacle (31),
- wherein the cartridge holder (20) comprises a first through recess (24) in a sidewall (22) of the insert section (21) which through recess (24) is configured to receive the first latch element (34), wherein the through recess (24) comprises a narrowed neck receiving portion (24a) extending axially into a widened latch receiving portion (24b), wherein a first width (w1) of the neck receiving portion (24a) is smaller than a second width (w2) of the latch receiving portion (24b).

2. The housing according to claim 1, wherein the first through recess (24) adjoins the proximal end (23) of the cartridge holder (20) and wherein the through recess (24) is open towards the proximal end (23).

3. The housing according to claim 1 or 2, wherein the neck receiving portion (24a) adjoins the proximal end (23) and wherein the neck receiving portion (24a) is located axially between the latch receiving portion (24b) and the proximal end (23).

4. The housing according to any of the preceding claims, wherein the first latch element (34) comprises a narrowed neck portion (34a) extending axially into a widened latch portion (34b).

5. The housing according to claim 4, wherein the latch portion (34b) comprises a maximal width (w3) that is larger than the first width (w1).

6. The housing according to claim 5, wherein the latch portion (34b) of the first latch element (34) is wedge-shaped with an increasing width in proximal direction (2).

7. The housing according to claim 5 or 6, wherein the latch portion (34b) comprises a minimal width (w4) at a distal end and further comprises the maximal width (w3) at a proximal end.

8. The housing according to any one of the preceding claims, wherein the receptacle (31) comprises a first protrusion (134) which extends inwardly from the sidewall (32) of the receptacle (31) at a first distance (t1) from the first latch element (34).

9. The housing according to claim 8, wherein the insert section (21) comprises a first slit (124) adjoining the proximal end (23) and extending in distal direction (1) from the proximal end (23) at the first distance (t1) from the first through recess (24) and wherein the first slit (124) is configured to receive the first protrusion (134).

10. The housing according to claim 9, wherein a first sidewall section (122) of the insert section (21), which is confined by the first through recesses (24) and the first slit (124), is flexible in a tangential direction from an initial unbiased state towards a biased state when the latch element (34) axially enters into the first through recess (24).

11. The housing according to claim 10, wherein a transverse size (S1) of the first protrusion (134) is substantially equal to a transverse size (S2) of the first slit (124) when the first sidewall section (122) is in the unbiased state.

12. The housing according to claim 10 or 11, wherein the first protrusion (134) comprises a lateral abutment face (135) to abut with a lateral abutment face (125) of the first sidewall section (122) of the insert section (21).

13. The housing according to any one of the preceding claims 8 to 12, wherein the first protrusion (134) comprises a ramp section (136) with a beveled surface (138) extending inwardly from the inside (35) of the sidewall (32) of the receptacle (31).

14. The housing according to claims 12 and 13, wherein the ramp section (136) is located at or near a proximal end of the receptacle (31) and wherein the lateral abutment face (135) of the first protrusion (134) adjoins the ramp section (136).

15. An injection device for administering an injectable medicament into biological tissue, the device comprising:
- a housing according to any one of the preceding claims,
- a drive mechanism (5) assembled inside the body (30), and
- a cartridge (50) assembled inside the cartridge holder (20).
